# EUROPÄISCHE PATENTANMELDUNG

(11) **EP 2 479 251 A1**
(43) Veröffentlichungstag der Anmeldung: **25.07.2012**
(21) Anmeldenummer: 12000425.4
(22) Anmeldetag: 24.01.2012
(51) Int. Cl.: C12G 1/02, C12N 1/18, C12M 1/00

(54) **Vorrichtung zum Rehydrieren von Mikroorganismen**

(30) Priorität: 25.01.2011 DE 202011001869 U
(71) Anmelder: Leo Kübler GmbH, 76133 Karlsruhe (DE)
(72) Erfinder: Alber, Bernd, Dr., 76227 Karlsruhe (DE); Wolf, Dieter, 76287 Rheinstetten (DE)
(74) Vertreter: Lempert, Jost

(57) **Zusammenfassung**

Die Erfindung betrifft eine Vorrichtung zum Rehydrieren von Mikroorganismen. Um eine solche einfach aufzubauen und preiswert zu gestalten, dennoch eine zuverlässige und sichere Reaktivierung der Mikroorganismen bewirken zu können, sieht die Erfindung vor, dass die Vorrichtung einen Behälter mit einer Temperaturregeleinrichtung, mit einem in den Behälter eingefüllte Flüssigkeit rührenden Rührer und mit einem Einfülltrichter zum sukzessiven Einfüllen pulverförmiger Mikroorganismen in den Behälter aufweist. Weiterhin sieht die Erfindung zur Lösung der genannten Aufgabe ein Verfahren vor, bei dem Flüssigkeit im Behälter auf eine gewünschte Temperatur aufgeheizt wird, bei sukzessivem rieselndem Einfüllen der Mikroorganismen, gegebenenfalls mit Mobilisator, in die im Behälter befindliche Flüssigkeit diese mittels eines Rührers über eine vorgegebene Zeit umgerührt wird, die Zieltemperatur über eine vorgegebene Zeit aufrecht erhalten wird, und anschließend unter Abschaltung der Heizeinrichtung wiederum ein akustisches Signal ausgegeben wird.

## Beschreibung

Die Erfindung betrifft eine Vorrichtung zum Rehydrieren von Mikroorganismen und zum Mischen von Bentonit mit Wasser.

Die Erfindung betrifft insbesondere die Rehydratation von Trockenhefe zwecks Reaktivierung derselben. Trockenhefe wird zum Vergären von Most oder Maische zu Wein oder Wein (zweite Gärung) eingesetzt. Bevor sie dem Most oder der Maische in einem Tank oder Weinflaschen zugesetzt wird, muss sie zunächst durch Rehydratation in Wasser oder in einem Most-Wasser-Gemisch reaktiviert werden, um dann nach Einfüllen der so gebildeten Hefesuspension in einen Most- oder Maischetank oder Flaschen ihre Gärwirkung zu entfalten.

Darüber hinaus betrifft die Erfindung auch die Rehydrierung von - gefriergetrockneten - Bakterien ebenfalls um diese zu reaktivieren. Diese dienen zum biologischen Säureabbau in Most oder Wein. Auch hier muss zunächst mit Wasser eine Suspension der fraglichen Bakterien hergestellt werden, die dann dem Most zugegeben wird und während der Gärung Apfelsäure in Milchsäure verwandelt, um auch einem durchgegorenen Wein einen übermäßigen Säurecharakter zu nehmen.

Weiterhin betrifft die Erfindung auch das Mischen von Bentonit mit Wasser (zur Eiweißstabilisierung des Weins).

Die Rehydratation von Trockenhefe und das Mischen von Bentonit erfolgt in den meisten Betrieben in aufwändiger, insbesondere zeitaufwändiger Weise von Hand, indem übliche Gerätschaften, z.B. Schneebesen, eingesetzt werden. Darüber hinaus ist aus der EP 2 055 768 A1 eine aufwändige, weitgehend automatisierte Vorrichtung zur Rehydratation von Trockenhefe bekannt, die aber aufgrund ihrer Kompliziertheit und ihrer Auslegung und den damit verbundenen Kosten nur zum Einsatz in Großbetrieben geeignet ist. Jedoch bietet auch diese Vorrichtung keine Hinweise zur Lösung des Problems des Verklumpens von Trockenhefe bei Herstellen der Trockenhefe-Wasser-Most-Suspension sowie darüber hinaus des Problems der übermäßigen Schaumbildung beim Einfüllen von Trockenhefe in ein Most-Wasser-Gemisch und derartige Herstellung einer entsprechenden Suspension.

Der Erfindung liegt daher die Aufgabe zugrunde, eine Vorrichtung zur Rehydratation von Mikroorganismen, insbesondere von Trockenhefe zu schaffen, die einfach aufgebaut und damit preiswert ist, dennoch eine zuverlässige und sichere Reaktivierung der Mikroorganismen bewirkt, ohne dass es zu Fehlern aufgrund von Unter- oder Übertemperatur oder falschen Zeitdauern kommt. Darüber hinaus sollen insbesondere die vorgenannten Probleme der Verklumpung der Trockenhefe und des Bentonit in der Suspension sowie der Schaumbildung bei der Produktion der Hefesuspension vermieden werden, da alle die genannten Probleme zu einer Schädigung der Mikroorganismen und bei Hefen zu unzureichender Gärung führen können.

Erfindungsgemäß wird die genannte Aufgabe gelöst durch eine Vorrichtung zum Rehydrieren von Mikroorganismen, mit einem Behälter mit einer Temperaturregelung, mit einem eine in den Behälter eingefüllte Flüssigkeit rührenden Rührer und mit einem Einfülltrichter zum sukzessiven Einfüllen pulverförmiger Mikroorganismen in den Behälter.

Mittels der erfindungsgemäßen Vorrichtung kann zuverlässig eine Mikroorganismen-, insbesondere eine Hefesuspension zur Mostvergärung und damit Weinherstellung hergestellt werden, wobei durch die erfindungsgemäße Ausgestaltung der Vorrichtung insbesondere eine Klumpenbildung der Hefe in der Suspension vermieden wird und eine vollständig gleichmäßige Suspension herstellbar ist.

In weiterer Ausgestaltung der Erfindung ist vorgesehen, dass sie ein Deckteil mit einem auf dem Behälter aufzusetzenden Deckel aufweist, wobei insbesondere unmittelbar unterhalb des Deckels ein Schaumzerstörer angeordnet ist. Dieser kann vorzugsweise als Rotor ausgebildet sein, weil insbesondere nach unten gerichtet, sich über die Länge des Rotors erstreckendes Blatt aufweist, das eine einem Rotorbalken abgewandte Kante aufweist. Auch wenn Rührer und Schaumzerstörer vom gleichen Antrieb angetrieben werden, so dreht sich der Rotor mit einer erheblich höheren Geschwindigkeit als der Rührer, vorzugsweise mit der 10-fachen Geschwindigkeit des Rührers. Durch den Rotor wird bei der Bildung einer Hefe-Most-Wasser-Suspension durch die einsetzende Gärung aufsteigender Schaum zerteilt und unterdrückt und damit der Austritt von Schaum aus dem Behälter verhindert.

In bevorzugter Ausgestaltung kann weiterhin vorgesehen sein, dass der Schaumzerstörer (der Mikroorganismen) als Durchbrechungen aufweisende Scheibe ausgebildet ist.

In bevorzugter Weiterbildung ist vorgesehen, dass der Schaumzerstörer einzelne Rotorblätter aufweist, die vorzugsweise gegen die Ebene des Schaumzerstörers geneigt sind und damit unter einem endlichen Winkel ungleich 90° einer Drehachse des Schaumzerstörers ausgerichtet sind, wobei der Anstellwinkel zur Achse vorzugsweise zwischen 5° und 10° beträgt.

In bevorzugter Ausgestaltung ist dabei vorgesehen, dass ein Rührer einen Schwimmbalken aufweist, der drehfest, aber axial verschiebbar auf einer Antriebswelle gelagert ist, wobei insbesondere der Schwimmbalken aus einem Material mit einer Dichte zwischen 0,9 und 0,95 g/cm³ besteht. Das Material ist vorzugsweise Polypropylen oder Polyethylen, wobei sich bei letzterem insbesondere LD- oder LLD-Polyethylen empfiehlt. Der Schwimmbalken kann zur Verringerung der Dichte mit Styropor oder anderen Materialien mit geringer Dichte gefüllt sein. Damit nichts von den pulverförmigen Materialien sich auf der Oberfläche des Drehbalkens dauerhaft festsetzt, kann der Rührbalken aus wasserabweisendem Rohr (z.B. aus PTFE), gefüllt mit Styropor, sein. Äußere Abschnitte des Balkens können dazu auch axial, d.h. um ihre Achsen, drehbar sein.

Weitere bevorzugte Ausgestaltungen sehen eine Dosiereinrichtung zum Eindosieren der Mikroorganismen vor, wobei insbesondere die Dosiereinrichtung eine gelochte Dosierscheibe und unmittelbar oberhalb sowie unterhalb derselben mit radial erstreckender Einlass- bzw. Auslassscheibe versehene Einlass- und Auslassscheiben aufweist und/oder die Schlitze der Einlass- und Auslassscheibe unter einem ersten Winkel α in Umfangsrichtung zueinander versetzt sind, die Schlitze der Auslassscheibe zu den Schlitzen der Einlassscheibe um einen zweiten Winkel β = α/2 in Umfangsrichtung zueinander versetzt sind und die Dosierscheibe unter dem Winkel β zumindest in Umfangsrichtung zueinander versetzt Dosierlöcher aufweist. Alternativ kann eine Siebeinheit zum Einfüllen des pulverförmigen Materials (der Mikroorganismen) in den Behälter vorgesehen sein, wobei die Siebeinheit zwei übereinander angeordnete relativ zueinander verschwenkbare Siebe aufweist.

Gemäß einer bevorzugten Weiterbildung ist vorgesehen, dass mindestens einer der Rührbalken beidseits eines Mittelteils relativ zu diesem um ihre Achse drehbar gelagerte Drehabschnitte aufweist, wobei insbesondere die Drehabschnitte in ihrer Seitenwandung sich unter einem endlichen Winkel zur Drehachse erstreckende Nuten (der Mikroorganismen) aufweisen. Weiterhin kann vorgesehen sein, dass der Rührer zwei Rührbalken aufweist, von denen einer auf der Flüssigkeit schwimmt, ein weiterer axial festgehaltener in die Flüssigkeit eintaucht.

Bei Antrieb von Rührer und Schaumzerstörer über das gleiche Getriebe ist in bevorzugter Ausgestaltung weiterhin vorgesehen, dass Rührer und/oder Schaumzerstörer über ein Unter- oder Übersetzungsgetriebe mit dem Antrieb verbunden sind. Weiter bevorzugte Ausgestaltungen sehen vor, dass der Einfülltrichter mit einem Rüttelantrieb (der Mikroorganismen) versehen ist, dessen Rüttelfrequenz vorzugweise einstellbar ist und/oder durch eine Siebeinheit zum Einfüllen des pulverförmigen Materials (der Mikroorganismen) in den Behälter, wobei insbesondere die Siebeinheit zwei übereinander angeordnete, relativ zueinander verschwenkbare Siebe aufweist. Demgemäß sieht die Erfindung verfahrensmäßig vor, dass das Suspensionsmaterial über zwei Siebe in den Behälter eingefüllt wird, dessen eines relativ zum anderen verschwenkt wird.

Insbesondere bei einer Ausgestaltung der erfindungsgemäßen Vorrichtung mit einem über ein Getriebe angetriebenen Rührer sieht die Erfindung weiterhin vor, dass im Bodenbereich ein Lager für die Antriebswelle des Rührers vorgesehen ist. Dieses kann in einer über dem Boden eingespannten Feder ausgebildet sein.

Eine weitere bevorzugte Ausbildung der Erfindung sieht vor, dass der Behälter eine Kühleinrichtung aufweist, die insbesondere mit einem innerhalb der Wandung des Behälters angeordnetes von Flüssigkeit durchströmbares Kühlgitter versehen ist.

Während sich grundsätzlich gezeigt hat, dass die Hefe-Suspension auch mit der zu ihrer Herstellung notwendigen und demgemäß bei der Herstellung der Suspension erzeugten Temperatur von 39°C unmittelbar in den Most eingefüllt werden kann, insbesondere wenn dies erfindungsgemäß über eine mit einer Pumpe versehenen vom Behälter zum Gärtank führenden Leitung und nicht manuell geschieht, kann die erfindungsgemäße Vorrichtung auch eine Kühlung aufweisen und zwar insbesondere für den Fall, dass eine Abkühlung der Hefe-Suspension auf eine niedrigere Temperatur gewünscht wird, die insbesondere nicht 10°C über der Most- bzw. Maische-Temperatur im entsprechenden Tank liegen soll. Dabei weist die Kühleinrichtung insbesondere ein innerhalb der Wandung des Behälters angeordnetes, Flüssigkeit durchströmbares Kühlgitter auf.

Durch die Temperaturregelung, die einen Temperaturfühler umfasst, kann einerseits die Temperatur des Mostes geregelt werden und zwar zum einen durch Regeln einer vorgesehenen Heizung, zum anderen auch durch Einwirkung auf den Kühlkreislauf. Andererseits kann die Regelung ein akustisches und gegebenenfalls auch optisches Signal ausgeben, wenn beispielsweise beim Aufheizen der im Behälter befindlichen Flüssigkeit auf die angestrebte Zieltemperatur (bei Hefereaktivierung 39°C, bei BSA-Bakterien ca. 25°C, beim Einrühren von Bentonit in Wasser ca. 60°C) diese erreicht wird und gegebenenfalls auch beim Abkühlen der Hefe-Suspension auf eine Einfülltemperatur in den Most bzw. die Maische die entsprechende Temperatur (nicht mehr als 20°C) erzielt wird. Entsprechendes gilt auch bei Ablauf der Verweilzeiten.

In bevorzugter Ausgestaltung ist vorgesehen, dass die Vorrichtung ein Deckteil mit einem auf dem Behälter aufzusetzenden Deckel aufweist, wobei sie weiterhin durch eine Schienenführung für das Deckteil zum Absenken desselben auf den Behälter unter Herstellung einer elektrischen Verbindung zur Leitungsversorgung und/oder einer Steuerverbindung und zum Abheben von diesem unter Trennung der elektrischen Verbindungen ausgebildet sein kann.

Die Erfindung sieht darüber hinaus ein Verfahren zum Reaktivieren von Mikroorganismen, insbesondere zur Rehydratation von Trockenhefe unter Einsatz der erfindungsgemäßen Vorrichtung vor, wobei Flüssigkeit, entweder Wasser und/oder ein Most-Wasser-Gemisch im Behälter auf eine gewünschte Reaktivierungs-/Rehydratations-Temperatur (in der Regel 39°C) aufgeheizt wird, bei Erreichen der genannten Temperatur ein akustisches Signal ausgegeben wird, anschließend bei sukzessivem rieselndem Einfüllen der Mikroorganismen und gegebenenfalls biologischem Mobilisator in die im Behälter befindliche Flüssigkeit diese mittels eines langsam umlaufenden Rührers über eine vorgegebene Zeit, insbesondere die Einfüllzeit der Mikroorganismen umgerührt wird, um eine Klumpenbildung zu vermeiden und die Zieltemperatur über eine weitere vorgegebene Zeit (in der Regel 25 Minuten) aufrecht erhalten wird sowie anschließend unter Abschaltung der Heizeinrichtung wiederum ein akustisches Signal ausgegeben wird.

In Weiterbildung kann vorgesehen sein, dass insbesondere bei einem Most-Flüssigkeitsgemisch, in welches die Mikroorganismen eingegeben werden, entstehender Schaum durch einen schnell laufenden mit einer Kante versehenen Rotor zerteilt und damit zerstört wird.

In Weiterbildung ist vorgesehen, dass die erzeugte Mikroorganismen-Suspension, insbesondere Hefe-Suspension, nach Erreichen einer vorgegebenen Haltezeit auf ebenfalls vorgegebener erhöhter Temperatur mittels einer Kühleinrichtung auf eine zweite vorgegebene Temperatur abgekühlt wird und nach Erreichen der vorgewählten zweiten Temperatur ein akustisches Signal ausgegeben wird. Hierauf kann dann die Suspension in einen Most- oder Wein enthaltenden Tank umgefüllt werden, wie von Hand.

Eine Weiterbildung der Erfindung sieht allerdings vor, dass die erstellte Suspension von einer Vorrichtung zur Erzeugung der Suspension mittels einer Pumpe durch eine Leitung in einen Most oder Maische enthaltenden Gärtank gepumpt wird.

Mittels der erfindungsgemäßen Vorrichtung kann neben der Rehydratation von Trockenhefe ebenfalls die Reaktivierung von Bakterien zum biologischen Säureabbau durchgeführt werden, wobei die Bakterien im Most Apfelsäure in Milchsäure umwandeln, um den Säurecharakter des herzustellenden Weins zu reduzieren. Hierbei werden getrocknete Bakterien, gegebenenfalls gefriergetrocknete Bakterien, in gleicher Weise in die erfindungsgemäß vorgesehene Vorrichtung, allerdings bei einer Temperatur im Bereich von 18 bis 20°C eingefüllt, die über 20 Minuten zur Rehydratation und damit Reaktivierung der Bakterien aufrecht erhalten wird. Anschließend kann die so hergestellte Suspension ebenfalls in den Most enthaltenden Gärtank umgefüllt werden, wobei dies ebenfalls wiederum mittels einer mit einer Pumpe versehenen Flüssigkeitsleitung geschehen kann.

Durch die erfindungsgemäße Vorrichtung, die vorzugsweise mit einem Behälter in der Größenordnung von bis zu 30 Liter Füllvermögen arbeitet, kann eine Mikroorganismen-Suspension für 15 bis 20 Kubikmeter Most bzw. Wein hergestellt werden, wodurch diese Füllmenge fast alle der in Europa üblicherweise verwendeten Gärtanks abgedeckt wird. Vorrichtungen mit größeren Behältern liegen ebenfalls im Rahmen der Erfindung, abgesehen davon, dass für größere Tanks mehrfach Suspensionen hergestellt werden können.

Schließlich kann die erfindungsgemäße Vorrichtung zur Herstellung einer klumpenfreien Bentonit-Suspension insbesondere aus Bentonitpulver oder aber auch aus Granulat hergestellt werden, die ebenfalls in Most oder Maische in einem Gärtank zur Weinherstellung eingefüllt wird. Gequollener Bentonit dient zur Schönung des Weins, um Weine zuverlässig gegen Eiweißtrübung zu schützen. Bei höheren Temperaturen (bis max. 60°C) verläuft die Quellung maximal schnell.

Darüber hinaus kann der Behälter der erfindungsgemäßen Vorrichtung auch zu weiteren Zwecken eingesetzt werden, wie beispielsweise aufgrund der Einrichtung mit Temperaturregelung zur Glühweinherstellung, so dass Zweitbehälter für diesen Zweck erspart werden können.

Weitere Vorteile und Merkmale der Erfindung ergeben sich aus den Ansprüchen und aus der nachfolgenden Beschreibung, in der Ausführungsbeispiele der Erfindung unter Bezugnahme auf die Zeichnung im Einzelnen erläutert sind. Dabei zeigt bzw. zeigen:
- Fig. 1: in schematischer Schnittdarstellung eine erste Ausführungsform der erfindungsgemäßen Vorrichtung;
- Fig. 2: eine schematische Darstellung einer Kühleinrichtung zum Einsatz in der erfindungsgemäßen Vorrichtung;
- Fig. 3: eine andere Ausgestaltung der erfindungsgemäßen in schematischer Darstellung;
- Fig. 3a: einen Rotor zur Schaumverhinderung bei der Ausgestaltung der Fig. 3 im Querschnitt;
- Fig. 4: eine schematische Schnittdarstellung durch das Getriebe zwischen Rotor und Rührer der Ausgestaltung der Fig. 3;
- Fig. 5: eine detaillierte Darstellung einer erfindungsgemäßen Vorrichtung;
- Fig. 6: eine Seitenansicht einer weiteren Ausgestaltung der erfindungsgemäßen Vorrichtung mit angehobenem Deckteil;
- Fig. 7: einen Längsschnitt durch die Ausgestaltung der Fig. 6;
- Fig. 8: eine perspektivische Sicht auf den Einfülltrichter mit Rüttelantrieb;
- Fig. 9: eine Seitenansicht der wesentlichen Teile des Deckteils;
- Fig. 10: eine perspektivische Draufsicht auf diverse Elemente (Schaumzerstörer, Rührbalken und Antrieb) ohne Einfülltrichter;
- Fig. 11: eine Sicht auf Riemenantrieb und einen scheibenförmigen Schaumzerstörer;
- Fig. 12: eine Draufsicht von unten auf einen Teil der erfindungsgemäßen Vorrichtung mit einem Schaumzerstörer mit zur Drehachse unter einem endlichen Winkel ungleich 90° geneigten Rotorblättern des Schaumzerstörers;
- Fig. 13a-13c: eine weitere alternative Antriebsausgestaltung und -übersetzung;
- Fig. 14: eine schematische Explosionsdarstellung einer Dosiereinrichtung der erfindungsgemäßen Vorrichtung als Alternative einer Siebeinheit;
- Fig. 15: eine Draufsicht auf eine Dosierscheibe der Dosiereinheit der Fig. 13; und
- Fig. 16: eine Draufsicht auf eine Einlass- bzw. Auslassscheibe einer Dosiereinheit der Fig. 12 mit Einlass- bzw. Auslassschlitzen.

Die erfindungsgemäße Vorrichtung 1 weist einen Behälter 2 auf. Ein solcher Behälter hat ein Fassungsvermögen von 27 Liter, wobei die Höhe zwischen etwa 40 bis 50 cm, und der Durchmesser zwischen 30 bis 40 cm liegt. Auf diesem ist im dargestellten Ausführungsbeispiel ein Deckteil 3 aufgesetzt. Das Deckteil 3 weist eine Trichteraufnahme 4 zur Aufnahme eines Trichters 5 auf. Weiterhin ist im Deckteil 3 ein Rührer 6 mit einer Antriebswelle 6 gelagert, die einen von Kreisform abweichenden Querschnitt, beispielsweise ähnlich eines Ovals aufweist, auf der ein Rotor oder Schwimmbalken 7 derart drehfest, aber axial beweglich, aufsitzt. Der Schwimmbalken 7 besteht aus einem Material mit geringerem spezifischem Gewicht als Wasser, so dass er in einer in dem Behälter eingefüllten Flüssigkeit 8 (Wasser) schwimmend eintaucht. Das spezifische Gewicht liegt vorzugsweise zwischen 0,9 und 0,95 g/cm³. Als Material kommt insbesondere Polypropylen oder LD- bzw. LLD-Polyethylen in Frage. Der Rotor kann auch aus wasserabweisendem Material, z.B. aus PTFE-Rohr, gefüllt mit Styropor, sein. Die Welle 6 ist in einem Lager 6.1 in einem Boden 3.1 des Deckteils 3 gelagert und wird über einen Antrieb 6.2 (Motor und gegebenenfalls Getriebe) angetrieben.

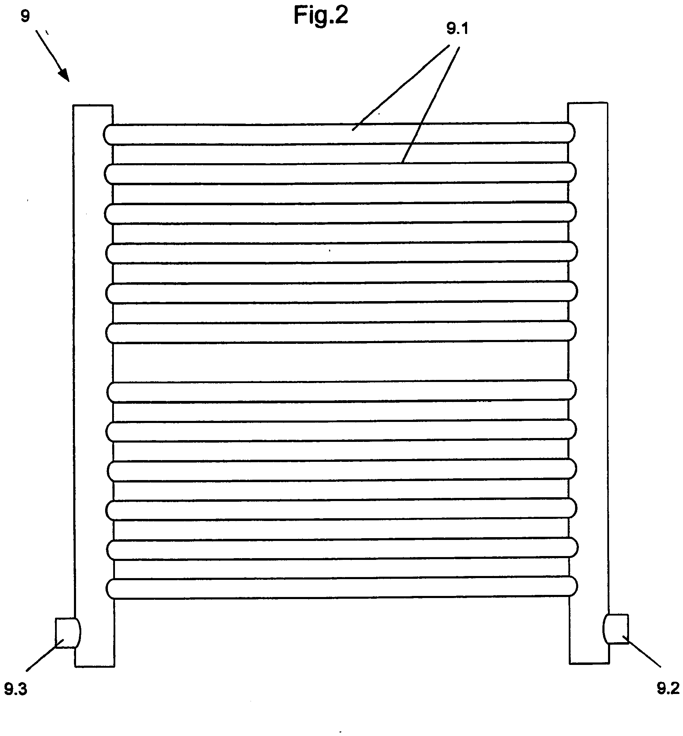

Im dargestellten Ausführungsbeispiel ist auf der Innenseite der zylindrischen Behälterwand 2.1 eine Kühlung 9 angeordnet, die beispielsweise als durch eine Flüssigkeit durchströmbares Kühlgitter 9.1 ausgebildet ist, wie dies in der Fig. 2 dargestellt ist. Durch das Kühlgitter kann Flüssigkeit (Wasser) von einem Einlass 9.2 aus zu einem Auslass 9.3 hindurchgeleitet werden.

An der Wandung 2.1 des Behälters 2 ist ein Elektroanschluss 2.2 vorgesehen. Im Boden 2.3 ist eine Elektronik bzw. Steuerung 10 mit einer Heizung 11 angeordnet. Der Steuerung ist ein Bedienfeld 10.1 zugeordnet. Unmittelbar über dem Boden ist ein Ablasshahn 2.4 vorgesehen.

Die Steuerung weist einen Regler für die Heizung 11 auf. Weiterhin ist mit der Steuerung ein Lautsprecher 10.2 verbunden, über den akustische Signale bei Erreichen vorgegebner Temperaturen ausgegeben werden.

Der Einsatz der erfindungsgemäßen Vorrichtung zur Reaktivierung von Hefe in Form von Rehydratation derselben ist folgendermaßen:
Zunächst wird in den Behälter 2 Wasser eingegeben. Anschließend wird über das Bedienfeld 10.1 die für die Eingabe der Hefe vorgesehene Temperatur von in der Regel 39°C vorgewählt und die Heizung 11 eingeschaltet. Bei Erreichen der voreingestellten Temperatur (39°C) gibt die Steuerung 10 über den Lautsprecher 10.2 ein Signal aus, mit dem das Erreichen der genannten Temperatur angezeigt wird. Der Nutzer der erfindungsgemäßen Vorrichtung 1, also im vorliegenden Fall ein Winzer oder Kellereiarbeiter, füllt in den Trichter 5 die gewünschte und gegebenenfalls auf die im Behälter befindliche Flüssigkeit abgestimmte Menge von Trockenhefe ein gemischt mit einem Hefemobilisator. Die Menge Trockenhefe beträgt in der Regel 15 bis 20 g/hl Most.
Gleichzeitig wird durch den Nutzer der Antrieb 6.2 für den Rührer 6 in Bewegung gesetzt, beispielsweise wodurch der Rührer 6 mit einer Umdrehungszahl von 15 bis 20 Umdrehungen pro Minute angetrieben wird.

Anschließend rieselt die Hefe durch die Trichtermündung 5.1 in den Behälter und damit in die Flüssigkeit 8. Das Einrieseln der Hefe in die Flüssigkeit dauert bei etwa halb gefülltem Behälter 5 bis 10 Minuten bei ca. 2,5 kg Hefe und ca. 2,5 kg Mobilisator. Der Rührer 6 bleibt über diese Zeit in Betrieb. Durch die Bewegung des Rührers wird ein Verklumpen der Hefe in der Flüssigkeit 8 verhindert und die Hefe in dieser fein dispergiert.

Nach vollständigem Einfüllen der Hefe in die Flüssigkeit 8 hält die Heizung 11 mittels ihres Reglers die eingestellte Temperatur von 39°C mit hoher Genauigkeit weiterhin über eine voreingestellte Zeit von in der Regel ca. 25 Minuten aufrecht. Nach dieser Zeit ertönt ein weiteres akustisches Signal. Nun kann der Winzer oder Kellereiarbeiter, falls gewünscht, die rehydratierte Hefeflüssigkeit in den der Hefegärung zu unterwerfenden Most oder die entsprechende Maische einfüllen.

Alternativ kann bei ausgeschalteter Heizung der Kühlkreislauf in Betrieb gesetzt werden, wobei eine Temperatur von nicht mehr als 20°C vorgewählt wird, die über ein vorgesehenes Thermometer oder einen Temperaturfühler (nicht dargestellt) und die Steuerung 10 überwacht wird. Nach Erreichen der voreingestellten Temperatur ergeht wiederum über den Lautsprecher 10.2 ein akustisches Signal, woraufhin dann die Hefeflüssigkeit in den Most bzw. die Maische umgefüllt werden kann, falls das Einfüllen der Hefeflüssigkeit bei einer Temperatur gewünscht wird, die nicht von der Ausgangstemperatur des Most bzw. der Maische abweicht, wobei die Temperaturdifferenz vorzugsweise nicht mehr als 10°C beträgt.

Das Umfüllen kann von Hand geschehen. Alternativ kann eine Schlauchleitung vorgesehen sein, die von der Vorrichtung 1 in den Most-/Maische-Tank führt und mit einer Pumpe versehen ist (nicht dargestellt), so dass die Hefeflüssigkeit aus dem Behälter 2 in den Most-/Maische-Tank umgepumpt werden kann.

Mit der erfindungsgemäßen Vorrichtung kann beispielsweise auch die Reaktivierung von Bakterien zum biologischen Säureabbau vorgenommen werden. Hierzu regelt die Steuerung 10 eine voreingestellte Temperatur von 20 bis 25°C über Heizung/Kühlung ein. Der im Behälter 2 eingegebenen Flüssigkeit 8 werden dann die zu reaktivierenden Bakterien eingegeben und über eine Zeit von 20 Minuten bei der genannten Temperatur reaktiviert. Anschließend wird das Gemisch ebenfalls in den Most bzw. die Maische eingegeben. Die Bakterien zum biologischen Säureabbau dienen bei der Weinherstellung dazu, Apfelsäure in Milchsäure umzuwandeln, um dem herzustellenden Wein bei weitgehender Durchgärung dennoch einen vorhandene Säurecharakter zu nehmen.

Schließlich kann die erfindungsgemäße Vorrichtung auch zum Einrühren von Bentonit in Pulver- oder Granulatform verwendet werden, wobei der Rührer 6 ebenfalls betätigt wird.

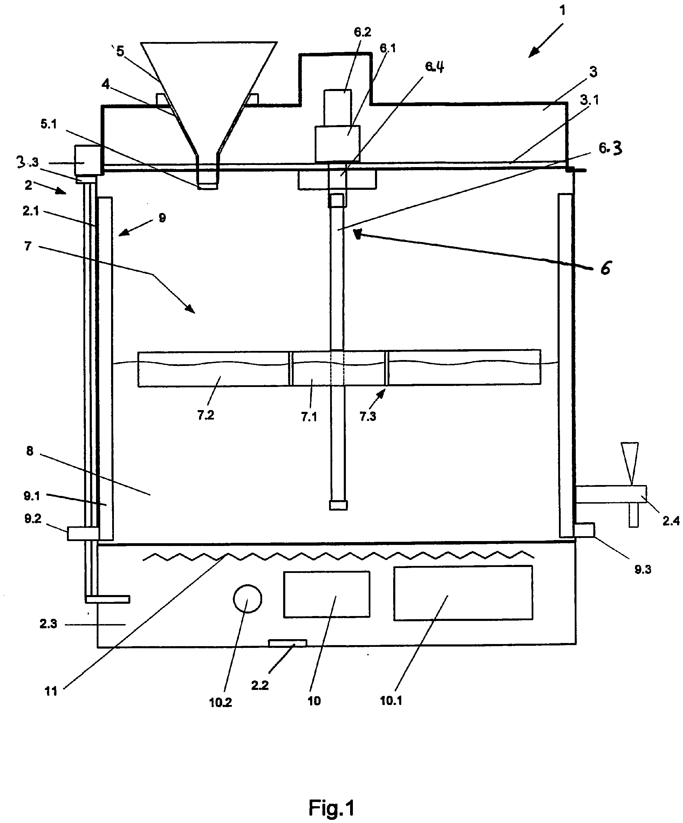

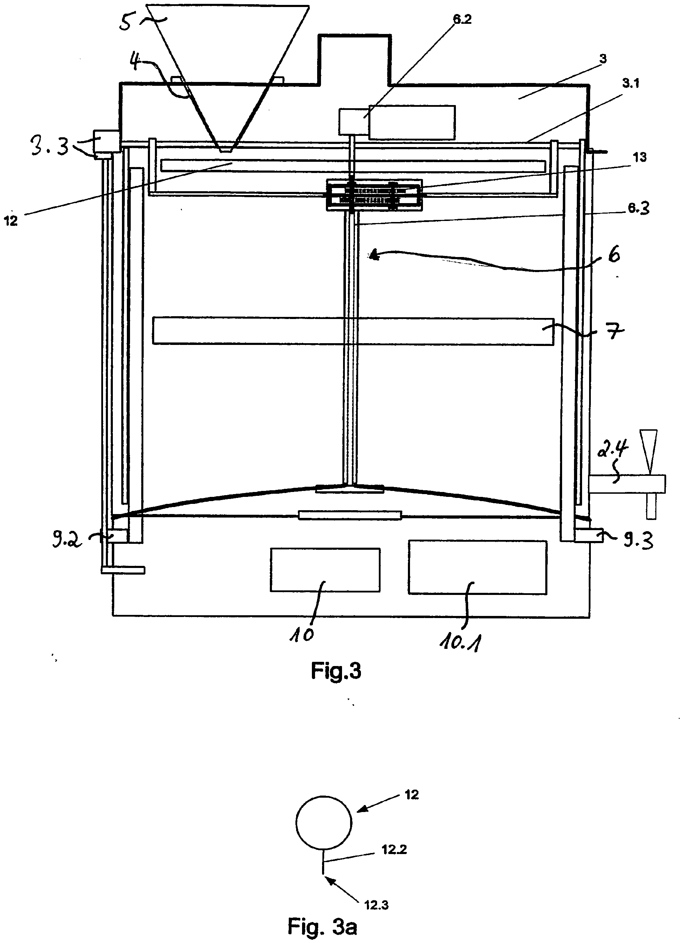

Die in der Fig. 3 dargestellte weitere Ausgestaltung der erfindungsgemäßen Vorrichtung weist zunächst den gleichen Grundaufbau auf, wie er unter Bezugnahme auf die Fig. 1 beschrieben wurde. Während bei der Ausgestaltung der Fig. 1 die Eingabe der Hefe und eines biologischen Hefe-Mobilisators (Hefenährstoffe) in reines Wasser erfolgt, wobei kein Schaum entsteht, wird es manchmal gewünscht, die Hefe in ein Most-Wasser-Gemisch einzugeben, wobei sogleich nach der Hydratation die Gärung einsetzt und zu einer erheblichen Schaumbildung (durch CO₂-Freisetzung) führt. In diesem Fall wird die Schaumbildung durch die Weiterbildung der erfindungsgemäßen Vorrichtung verhindert, wie sie in der Fig. 3 dargestellt ist. Soweit gleiche Teile vorhanden sind, sind diese mit gleichen Bezugszeichen versehen.

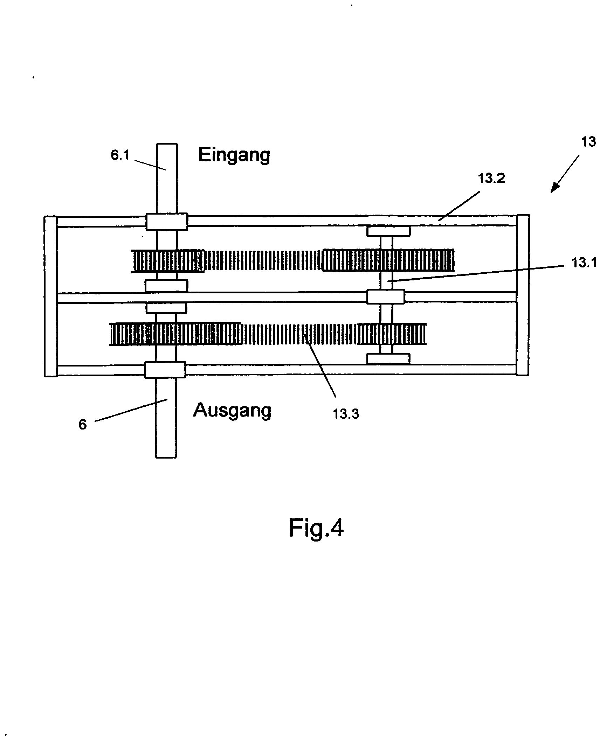

Unterhalb des Deckteils 3 und des Deckelbodens 3.1 ist bei der Ausgestaltung der Fig. 3 ein Schaumzerstörer in Form eines Rotors 12 vorgesehen, der aus einem im Querschnitt kreisförmigen Balken 12.1 mit einer nach unten gerichteten sich über die gesamte Länge des Rotors erstreckenden Blatt 12.2 mit einer scharfen Kanten 12.3 besteht (Fig. 3a). Dieser wird unmittelbar durch den Antrieb 6.2 angetrieben, und zwar mit einer relativ hohen Geschwindigkeit von 150 bis 200 Umdrehungen pro Minute. Um die Untersetzung der Umdrehungsgeschwindigkeit für den Rührer 6, der sich, wie gesagt, mit einer Umdrehungszahl von 15 bis 20 Umdrehungen pro Minute drehen soll, zu erzielen, ist zwischen dem Rotor und der Welle 6.3 des Rührers 6 ein Untersetzungsgetriebe 13 vorgesehen. Das Untersetzungsgetriebe 13, das in der Fig. 4 vergrößert dargestellt ist, weist als Eingangswelle 6.1 die Drehwelle des Rotors 12 als Abtriebswelle des Motors 6.2 und als Ausgangswelle die Drehwelle 6.3 des Rührers 6 auf. Das Getriebe ist als Riemengetriebe ausgebildet mit einer parallel zu den fluchtenden Wellen 6.1, 6.3 angeordneten Zwischenwelle 13.1. Im Getriebegehäuse 13.2 des Getriebes 13 sitzen auf den Wellen 6.1, 6.3 und der Zwischenwelle 13.1 entsprechend dimensionierte Riemenscheiben, über die Riemen 13.3 geführt sind, wodurch die Drehzahl von Eingangswelle 6.1 zur Ausgangswelle 6.3 im Verhältnis 10:1 reduziert wird (auf der Eingangswelle 6.1 sitzt eine kleine Riemenscheibe, eingangsseitig auf der Welle 13.1 eine mit demgegenüber größeren Durchmesser, ausgangsseitig auf der Welle 13.1 wieder eine kleine Riemenscheibe und auf der Welle 6.4 eine mit demgegenüber vergrößerten Durchmesser).

Der Ablauf der Rehydratation der Hefe ist im Wesentlichen der gleiche, wie er unter Bezug auf Fig. 1 beschrieben wurde. Durch den mit dem Blatt 12.2 und der unteren Kante 12.3 sich schnell drehenden Rotor 12 wird aufsteigender Schaum zerteilt, die Schaumbildung unterdrückt und damit die Ausbreitung von Schaum verhindert; entstandenes CO₂ kann nach Einfüllen der Hefe durch die Öffnung der Trichteraufnahme nach außen entweichen.

Die folgenden Figuren zeigen weitere Ausgestaltungen der erfindungsgemäßen Vorrichtung. Gleiche Teile sind jeweils mit gleichen Bezugszeichen versehen. Soweit bei einer folgenden Ausgestaltung Teile vorhanden sind, die bei einer vorherigen Ausgestaltung schon erläutert wurden, wird darauf verwiesen. Soweit im Übrigen zu einer Ausgestaltung Elemente dargestellt sind, in einer anderen nicht, so können diese auch bei der anderen Ausgestaltung mitverwirklicht sein.

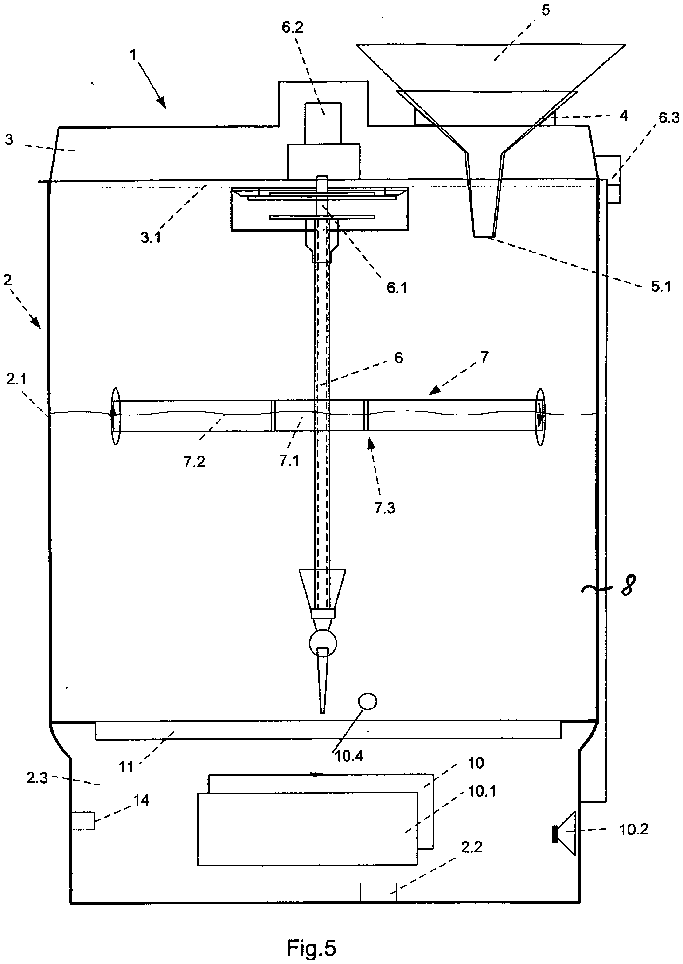

Die Fig. 5 zeigt insbesondere zwischen Behälter 2 und Deckel 3 einen Steckkontakt 3.3 zur Stromversorgung des Antriebs 6.2 vom Messanschluss 2.2, der im Boden 2.3 des Behälters angeordnet ist.

Bei der Ausgestaltung der Fig. 5 besteht der Schwimmbalken 7 aus einem Mittelteil 7.1 und zwei Außenteilen 7.2, die am Mittelteil 7.1 über jeweils ein Lager 7.3 drehbar gelagert sind. Hierdurch wird erreicht, dass sich beim Drehen der Welle 6 die Außenteile 7.2 des im Bereich der Oberfläche der Flüssigkeit 8 aufliegenden Rotors um ihre sich senkrecht zur Welle 6 erstreckende Achse in den Lagern 7.3 drehen und dadurch auf die obere Seite des Rotors 7 auffallenden Hefe und Aktivator in die Flüssigkeit 8 gebracht werden können und sich nicht auf der Oberseite des Schwimmbalkens 7 ansammeln.

Der Temperatursensor 10.4 ist über eine Leitung mit der Steuerelektronik 10 verbunden.

Weiterhin kann ein Elektroanschluss 14 für eine externe Pumpe vorgesehen sein.

Ansonsten wird auf die Beschreibung der vorhergehenden Ausführungsbeispiele, insbesondere die der Fig. 1 verwiesen. Bei der Ausgestaltung der Fig. 5 ist keine separate Kühlung vorhanden, da durch einen biologischen Mobilisator die Hefezellen so stabilisiert werden, dass es keinen Kälteschock gibt, wenn sie in den kühleren Most/Maische übergeben werden, so dass sie nicht vorab im erfindungsgemäßen Behälter 2 auf die Most/Maische-Temperatur abgekühlt werden müssen.

Zum Lieferumfang kann zusammen mit der erfindungsgemäßen Vorrichtung eine Waage zur genauen Bestimmung der für eine bestimmte Mostmenge erforderlichen Hefe- und Mobilisatormenge gehören. Die Zuordnung von Hefe und Mobilisator zu einer bestimmten Mostmenge kann für den Nutzer in verschiedener Weise erleichtert werden, beispielsweise durch einen Spezialrechner, der auch am erfindungsgemäßen Behälter integriert sein kann oder aber eine mitgelieferte Tabelle.

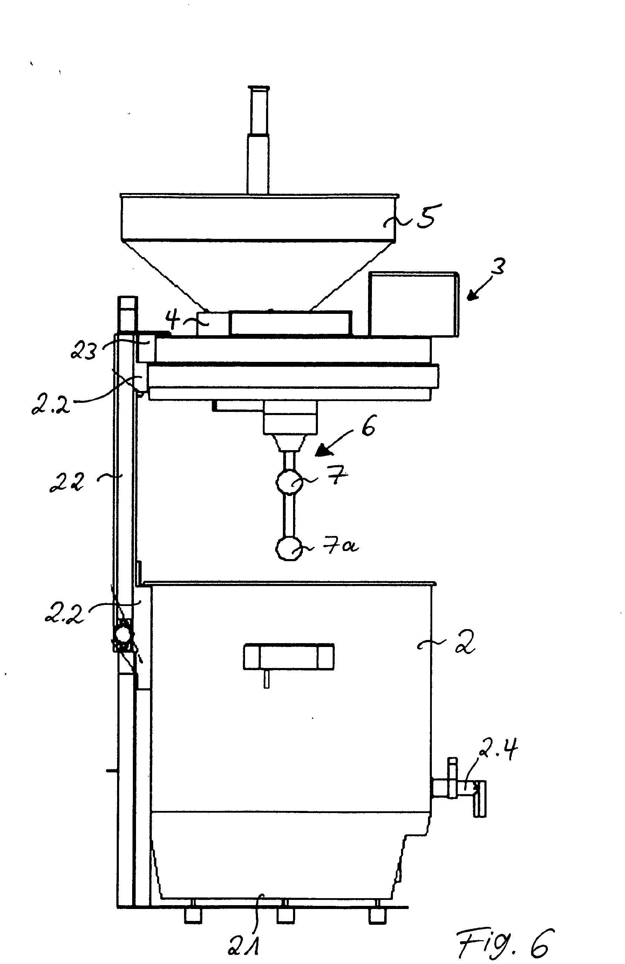

Bei der Ausgestaltung der Fig. 6 steht der Behälter 2 auf einer Plattform 21 und ist durch diese positioniert. Die Plattform 21 ist mit einer senkrechten Schienenführung 22 verbunden, an der das Deckteil 3 über ein Verbindungsteil 23 vertikal verschiebbar und damit auf den Behälter 2 absenkbar und von diesem anhebbar gelagert ist.

Der Rührer 6 weist nicht nur, wie oben beschrieben, einen ersten mit der Antriebsachse des Rührers 6 drehfest, aber axial beweglichen Rührbalken 7 auf, der so ausgebildet ist, dass er auf der Flüssigkeit im Behälter 2 schwimmt, sondern unter dem Rührbalken 7 einen zweiten Rührbalken 7a, der nicht nur dreh-, sondern auch axial fest mit der Antriebsachse des Rührers 6 verbunden ist und daher bei Absenken des Deckteils 3 in die im Behälter 2 befindliche Flüssigkeit tiefer eintaucht.

Am Verbindungsteil 23 und im Bereich der Oberseite des Behälters 2 an der Schienenführung 22 sind Steckverbindungen 2.2 ausgebildet, so dass hierüber von der Schienenführung 22 bei Absenken des Deckteils 3 auf den Behälter 2 ein elektrischer Kontakt zur Leistungsversorgung der im Deckteil 3 vorgesehenen elektrischen Elemente, wie die elektromotorischen Elemente, gegebenenfalls aber auch Sensoren, erfolgt.

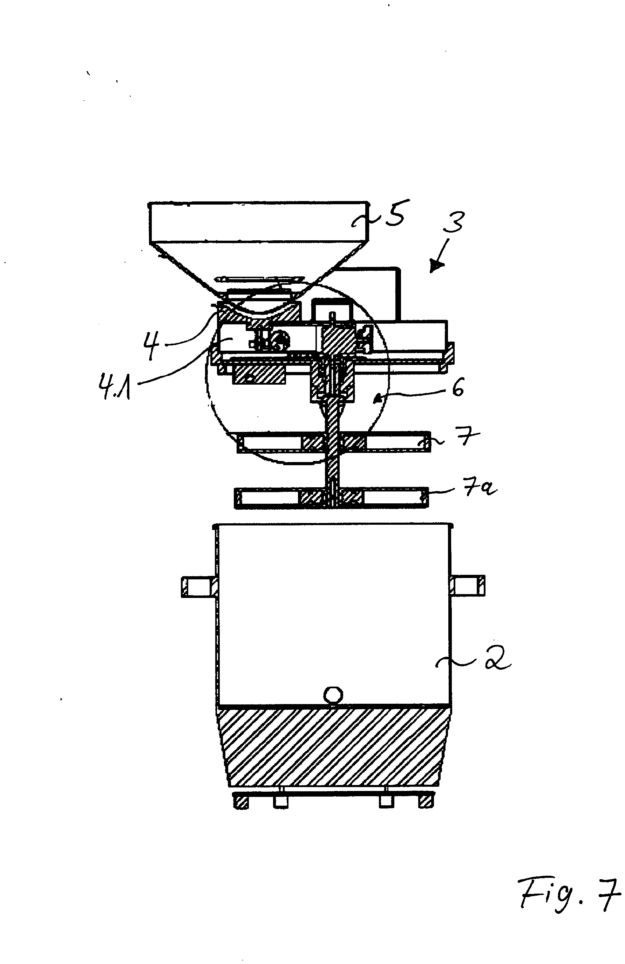

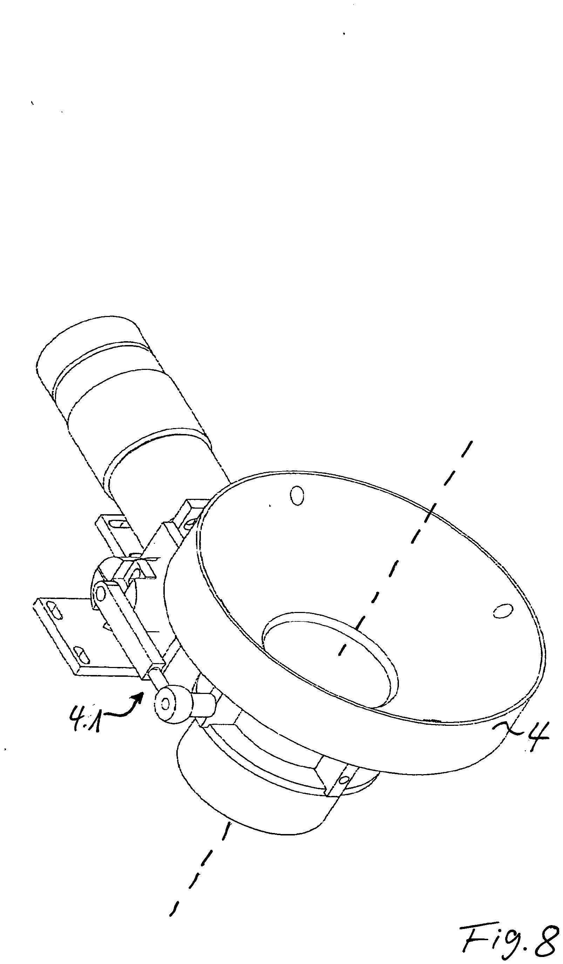

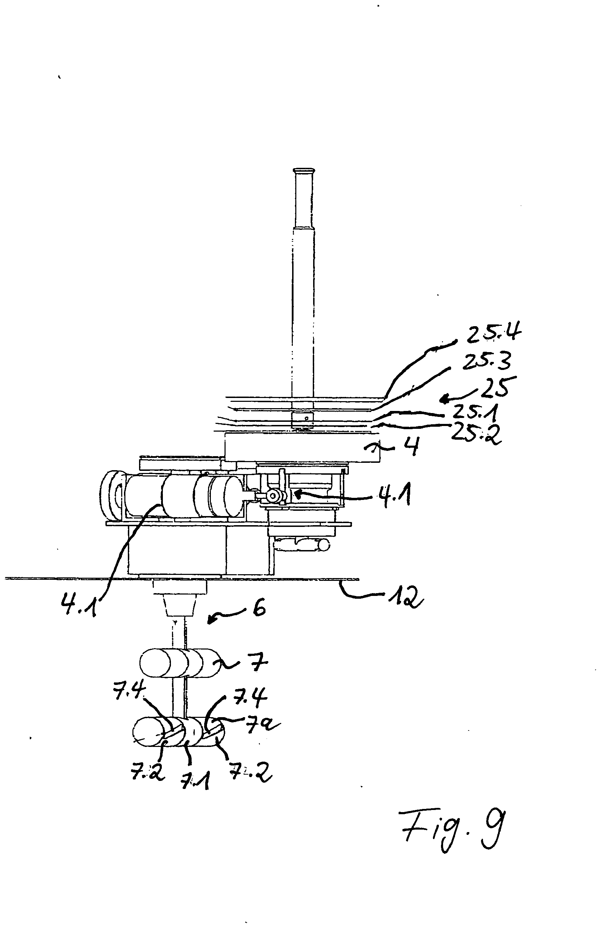

Die Fig. 7 zeigt Behälter 2 und Deckteil 3 in einem Längsschnitt, wobei ein Rüttelantrieb 4.1 für den Trichter 5 erkennbar ist, der auf die Rüttelaufnahme 4 wirkt. Die Fig. 8 zeigt den Einfülltrichter 4 mit Rüttelantrieb 4.1 zur Veranschaulichung in perspektivischer Darstellung. Es gibt Teile, auf die im Weiteren unter Bezugnahme, insbesondere auf die Fig. 9, eingegangen wird. Diese sind ebenso wie in Fig. 7 in auseinandergezogener Konfiguration dargestellt.

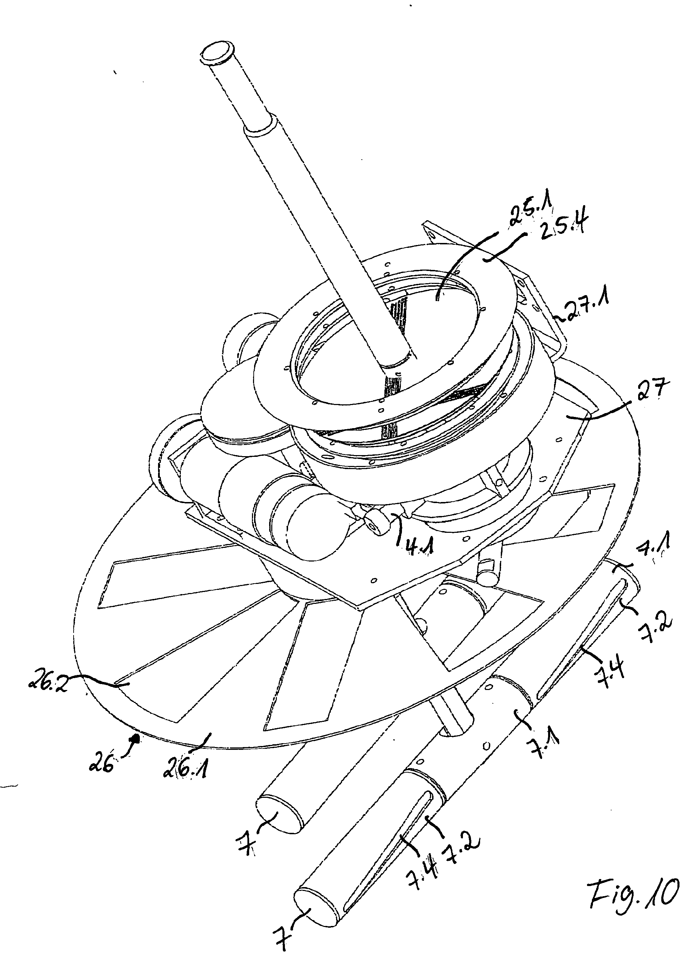

Wie auch der Fig. 10 zu entnehmen ist, weist die Siebeinheit 25 ein oberes Sieb 25.1 und ein unterhalb desselben befindliches Sieb 25.2 auf, die jeweils über einen Spannring 25.3 in einer Siebhalterung 25.4 festgelegt sind (für das untere Sieb 25.2 nicht dargestellt). Die Siebe 25.1, 25.2 sind über einen Antrieb relativ zueinander verschwenkbar.

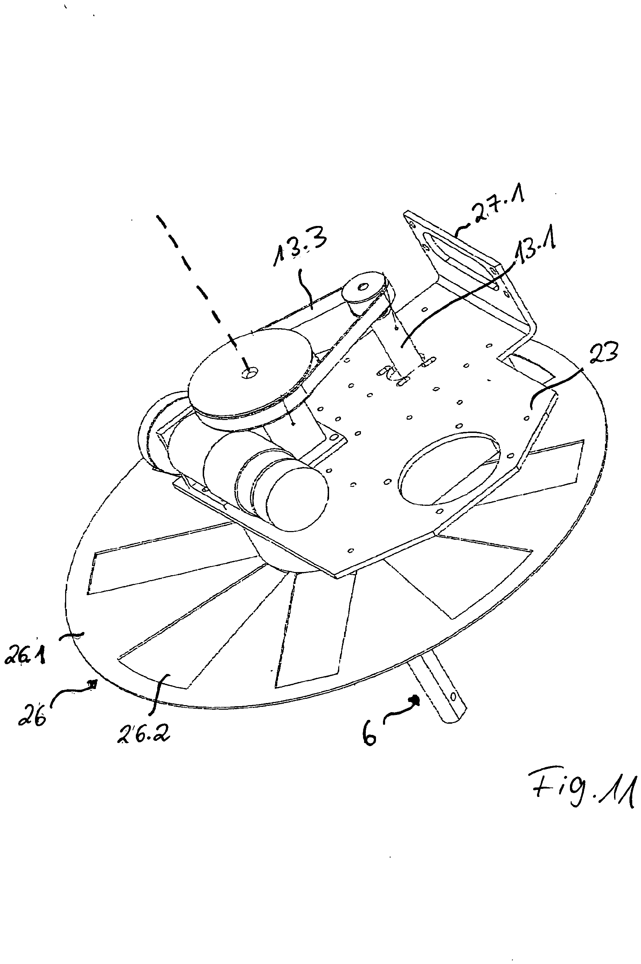

Wie insbesondere die Fig. 11 (aber auch die Fig. 10) zeigt, ist an der Unterseite des Deckteils 2 koaxial zur Hauptachse der erfindungsgemäßen Vorrichtung, die der Antriebsachse des Rührers 6 entspricht, ein Schaumzerstörer 26 in Form einer Scheibe 26.1 mit Durchbrechungen 26.2 angeordnet. Die Durchbrechungen 26.2 sind im Wesentlichen als sich radial erstreckende Schlitze in der Scheibe 26.1 ausgebildet.

Aus Fig. 9 ist weiterhin ersichtlich, dass dort der untere Rührbalken 7a in den Mänteln seiner drehbaren Außenteile 7.2 leicht schräg zu seiner Mittelachse verlaufende Nuten aufweist. Hierdurch wird beim Drehen des Rührbalkens 7a in die Antriebsachse des Rührers 6 eine Drehbewegung der drehbar gelagerten Außenteile 7.2 innerhalb der Flüssigkeit bewirkt und damit durch Mischung der herzustellenden Suspension unterstützt.

Mit 27 ist eine Montageplatte bezeichnet, die über einen Flansch 27.1 ein Verbindungsteil 23 zur Schienenführung 22 in das gesamte Deckteil 3 sowie die an diesem vorgesehenen Teile trägt.

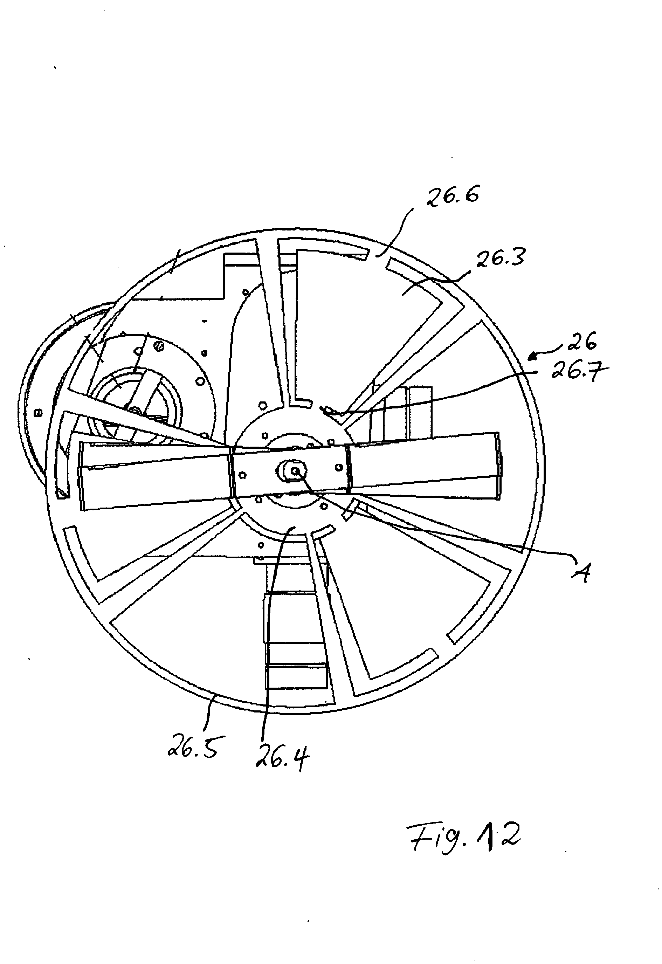

Die Fig. 12 zeigt eine alternative Ausgestaltung des Schaumzerstörers 26. Bei diesem sind Rotorblätter 26.3 am durch eine innere Scheibe 26.4 und einen äußeren Ring 26.5 gebildeten Hauptkörper des Schaumzerstörers 26 lediglich über zentrale Stege 26.6, 26.7 befestigt, während die Rotorblätter 26.3 ansonsten nicht mit dem Hauptkörper 26.4, 26.5 des Schaumzerstörers 26 verbunden sind. Dies bietet die Möglichkeit, die Rotorblätter 26.3 um die Verbindungslinie der Stege 26.6, 26.7 aus der Ebene des Schaumzerstörers 26 bzw. seines Hauptkörpers 26.4, 26.5 herauszukippen bzw. zu verkanten, so dass sie gegenüber dieser Ebene (die der Plattebene entspricht) um einen geringen Winkel von beispielsweise 5° bis 10° geneigt angeordnet sind. Dies bedeutet andererseits, dass die Rotorblätter 26.3 nicht senkrecht zur Drehachse A des Schaumzerstörers 26 (bzw. auch der Rotorbalken) gerichtet sind, sondern zur Rotorachse A einen endlichen Winkel ungleich 90° einschließen, nämlich einen Winkel zwischen 80° und 85°. Durch diese Verkantung bzw. Neigung der Rotorblätter 26.3 in der beschriebenen Weise wird die Schaumzerstörungswirkung des Schaumzerstörers 26 verbessert und zwar grundsätzlich unabhängig von der Drehrichtung, wobei in bevorzugter Weise die Drehrichtung allerdings so gewählt ist, dass die Rotorblätter 26.3 mit ihrer zur Flüssigkeitsöffnung der im Behälter befindlichen Flüssigkeit hingerichteten Kante gedreht werden, so dass diese in den entstandenen Schaum einschneidend diesen in der Drehbewegung zu ihrer rückwärtigen von der Flüssigkeitsoberfläche fortgerichteten Kante abhebt und damit die Schaumzerstörung verbessert. Zur Antriebsübersetzung des Schaumzerstörers 26 nach Fig. 10 wird auf die Ausgestaltung zu Fig. 4 verwiesen, die hier in gleicher Weise gelten. Die Übersetzung kann grundsätzlich durch eine Stufe - insbesondere die erste - bewirkt werden, so dass dann die Scheiben der weiteren Stufe gleichmäßig sind und eine 1:1-Übersetzung bewirken.

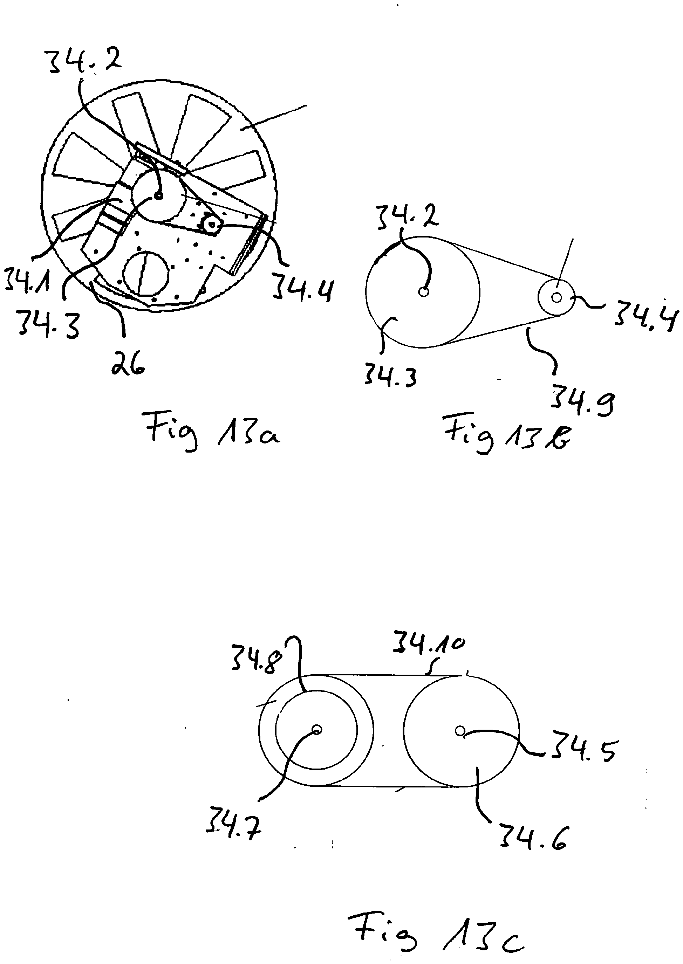

Eine zu der Antriebsausgestaltung 34 der Fig. 4 alternative Antriebsausgestaltung 34 ist in den Fig. 13a bis 13c dargestellt, wobei dieser Antrieb insbesondere für einen Schaumzerteiler oder -zerstörer in einer - mit Durchbrechungen versehenen Scheibe geeignet ist, wie sie insbesondere in den Figuren 10 bis 12 dargestellt ist.

Der Motorantrieb 34.1 weist ein Planetengetriebe auf, so dass die Ausgangsgeschwindigkeit des Motorantriebs 34.1 relativ langsam ist. Der Antrieb 34.1 greift über Kegelgetriebe (nicht dargestellt) an der Welle 34.2 für den Rührer 7 bzw. 7a an (Fig. 1, 3a, 5, 6, 7, 10). Auf der Antriebswelle 34.2 ist drehfest eine Riemenscheibe 34.3 mit relativ großem Durchmesser angeordnet, der eine kleine Zahnscheibe 34.4 zugeordnet ist. Diese ist drehfest mit einer weiteren Achse 34.5 verbunden, auf der wiederum eine relativ große Riemen- oder Zahnscheibe 34.6 sitzt, die etwa die gleiche Größe hat wie die Zahnscheibe 34.3. Auf einer Welle 34.7 für den Schaumzerstörer 26 ist - unterhalb der Plattebene der Fig. 13a - ebenfalls eine Zahnscheibe mit großem Durchmesser 34.8 drehfest angeordnet. Die Zahnscheiben 34.3 und 34.4 sind über einen Zahnriemen 34.9 und die Zahnscheiben 34.6 und 34.8 über einen Zahnriemen 34.10 miteinander verbunden.

Bei dieser Ausgestaltung wird also der Rührer 7, 7a durch den Motorantrieb 34.1 direkt mit langsamer Umdrehung angeordnet, während durch das beschriebene Zahnradgetriebe 34.3 bis 34.10 der Schaumzerstörer 26 mit einer höheren Geschwindigkeit antrieben wird. Das Übersetzungsverhältnis ist beispiels- und vorzugsweise 3.1.

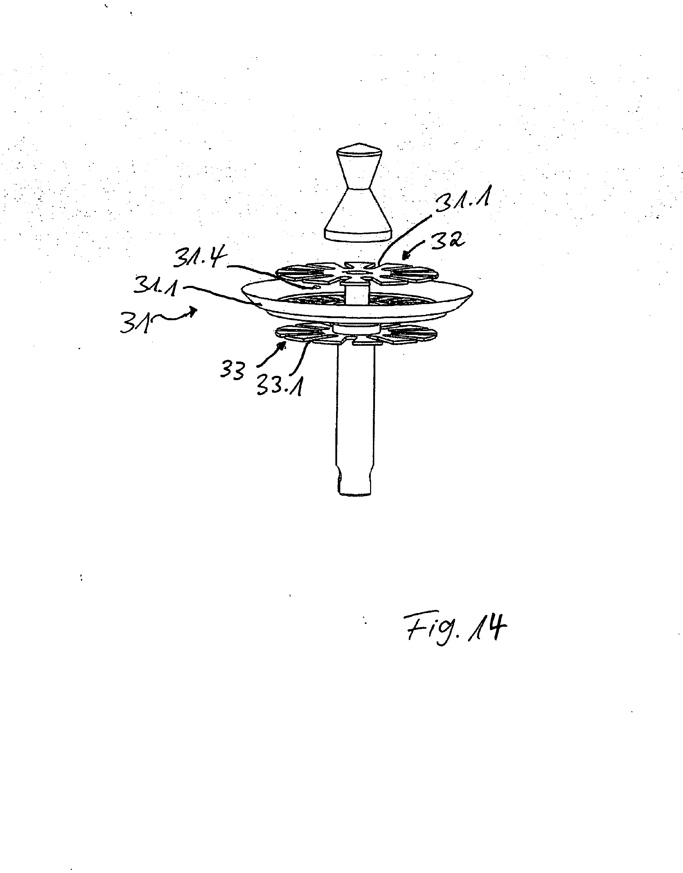

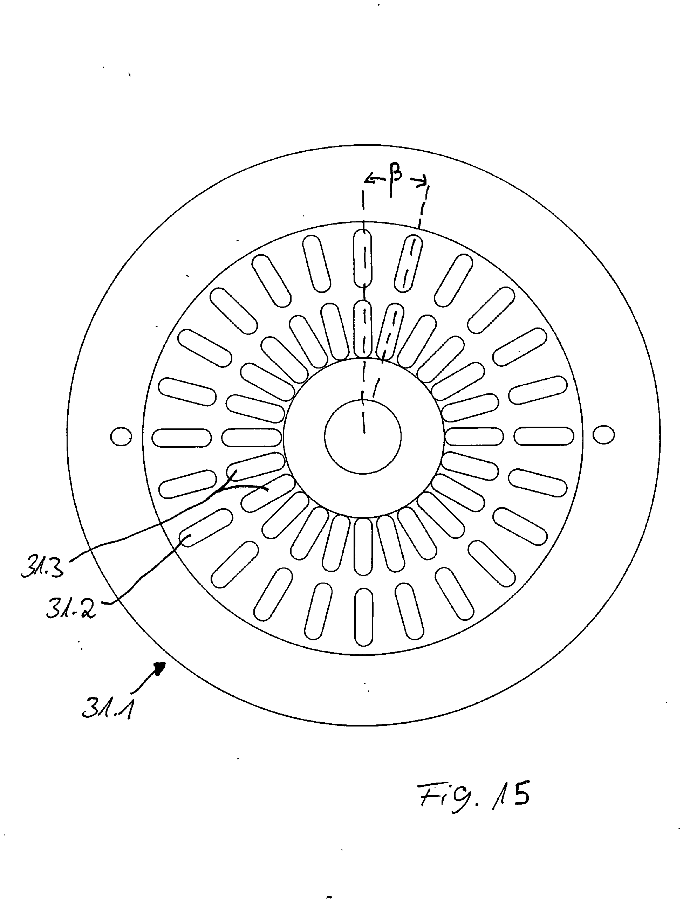

Alternativ zu der beschriebenen Siebeinrichtung zum Einsieben der Mikroorganismen in die im Behälter befindliche Flüssigkeit sieht eine äußerst bevorzugte Ausgestaltung statt der Siebeinrichtung eine Dosiereinrichtung 31 vor, wie sie in der Fig. 14 schematisch in Explosionsdarstellung dargestellt ist. Diese weist zunächst eine mit Langlöchern 31.2, 31.3 versehene Dosierscheibe 31.1 auf (Fig. 15). Die Langlöcher 31.2, 31.3 bilden Dosierkammern. Die Stärke der Dosierscheibe 31.1 in ihrem Bodenbereich und damit das Füllvolumen der durch die Länglöcher 31.2, 31.3 gebildeten Dosierkammern kann in gewünschter Weise ausgebildet sein, um eine geeignete Dosierung zu ermöglichen. Die Dosierscheibe 31.1 wird durch Verbindungslöcher 31.4 hindurchgesteckte Verbindungsstifte fest mit dem Dosiertrichter 5 bzw. der Trichteraufnahme 4 verbunden und wird demgemäß durch den Rüttelantrieb 4.1 mit angetrieben, wie dies oben beschrieben wurde.

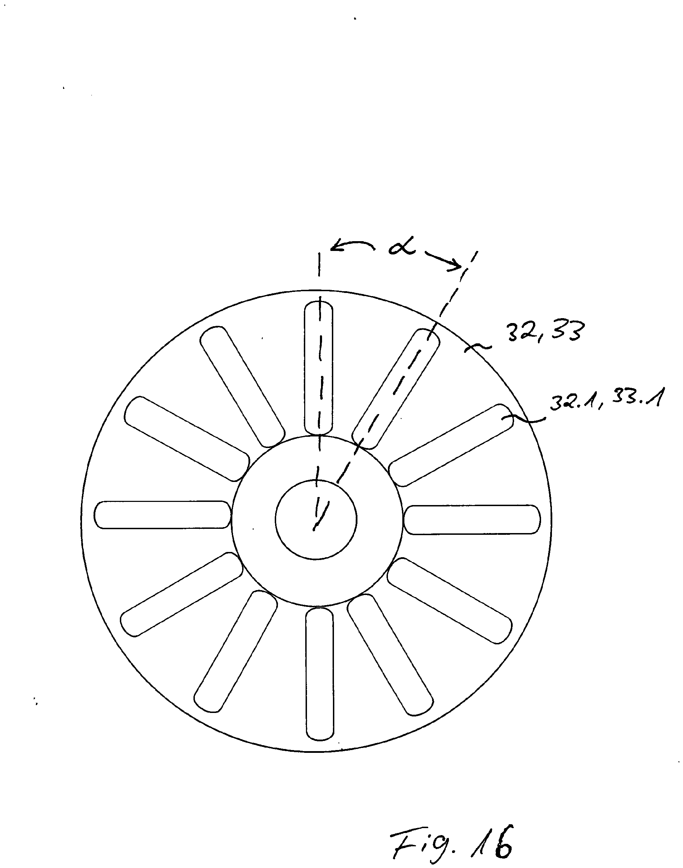

Oberhalb der Dosierscheibe 31.1 und unmittelbar auf dieser aufliegend ist eine Einlassscheibe 32 mit Einlassschlitzen 32.1 vorgesehen. Ebenfalls unmittelbar unterhalb der Dosierscheibe 31.1 und von unten an dieser unmittelbar anliegend ist eine Auslassscheibe 33 mit Auslassschlitzen 33.1 vorgesehen, wie sie in der Fig. 16 dargestellt sind. Einlass- und Auslassscheiben sind insofern identisch.

Die Einlassschlitze 32.1 bzw. 33.1 auf der Einlassscheibe 32 bzw. der Auslassscheibe 33 haben im Umfangsrichtung einen festen vorgegebenen über den gesamten Umfang hin gleichen Winkelabstand α, im vorgegebenen Beispiel der Fig. 15 von α = 30°.

Einlass- und Auslassscheibe 32, 33 sind drehfest miteinander verbunden, wobei ihre Schlitze 32.1, 32.3 allerdings zueinander um einen Winkel β = α 1/2 zueinander versetzt sind, also im dargestellten Ausführungsbeispiel um 15°, wie dies aus der Fig. 14 ersichtlich ist. Auch der Winkelabstand der sich radial erstreckenden Lochreihen der Dosierlöcher 31.2, 31.3 beträgt β = α/2, also im dargestellten Ausführungsbeispiel 15°.

Die Dosierscheibe 31.1 ist, wie gesagt, über den Rüttelantrieb 4.1 relativ zu den fest miteinander verbundenen Einlass- und Auslassscheiben 32, 33 bewegbar, nämlich verdrehbar. Der Rüttelantrieb 4.1 bewirkt ein Verdrehen oder eine Verschwenkung der Dosierscheibe 31.1 um einen Winkel β, im dargestellten Ausführungsbeispiel also 15°. Dies führt dazu, dass in der einen Endposition die jeweils jede zweite sich radial erstreckende Dosierlochreihe aus Dosierlöchern 31.2, 31.3 unmittelbar unter den Einlassschlitzen 32.2 der Einlassscheibe 32 zum Liegen kommt und damit durch die Einlassschlitze 32.1 der Einlassscheibe 32 Material - Mikroorganismen - in die Dosierlöcher 31.2, 31.3 hineinfallen kann. Durch den Antrieb 4.1 wird die Dosierscheibe 31.1 sodann um 15° verschwenkt, wobei die, wie vorstehend beschrieben, gefüllten Dosierlöcher 31.2, 31.3 über die Auslassschlitze 33.1 der Auslassscheibe 33 gelangen und damit das Material durch diese in den Behälter hineinfallen kann. In dieser Position ist die andere Hälfte der Dosierlöcher aufweisenden Dosierreihen aufgrund des genannten Winkelabstandes unterhalb der Einlassschlitze 32.1 angeordnet und kann befüllt werden und nach dem Zurückschwenken durch den Antrieb 4.1 in die Ausgangsposition kann das in dieser anderen Hälfte der Dosierlöcher eingefüllte Material durch die Auslassschlitze 33.1 der Auslassscheibe 33 ebenfalls in den Behälter hineinfallen.

Die Frequenz des Antriebs 4.1 kann in geeigneter Weise eingestellt werden und zwar in Abhängigkeit von Körnung, Rieselverhalten und gewünschtem Dosierdurchsatz, so dass ein geeignetes kontinuierliches Dosieren des Materials in der Flüssigkeit im Behälter möglich ist, ohne dass es zu Klumpenbildung etc. kommt.

Während der Antrieb 4.1 üblicherweise die genannten Verschwenkbewegungen um den Winkel β = α 1/2 ausführt, kann bei schlecht rieselfähigem Material nach rutschen desselben zwischen solchen Schwenkbewegungen über den Winkel β ein oder mehrere kurze Rüttelbewegungen mit einem wesentlich geringeren Verschwenkbereich ausgeführt werden, um die Dosierlöcher 31.2, 31.3 zu füllen, bevor dann das Verschwenken zum Entleeren der befüllten Dosierlöcher der Dosierscheibe 31.3 in der beschriebenen Weise erfolgt.

Bevorzugte Arbeitsgeschwindigkeiten bei der erfindungsgemäßen Vorrichtung und gemäß dem erfindungsgemäßen Verfahren liegen im folgenden Bereich:
Umdrehungsgeschwindigkeit des Rührers: 100 bis 150 Umdrehungen pro Minute, vorzugsweise 120 Umdrehungen pro Minute.
Umdrehungsgeschwindigkeit des Schaumzerteilers: 300 bis 450 Umdrehungen pro Minute, vorzugsweise 360 bis 400 Umdrehungen pro Minute.

Schwimmfrequenz des Rüttlerantriebs für den Einfülltrichter: 1/4 Hz bis 4 Hz, vorzugsweise bei 1 bis 2 Hz.

### Bezugszeichenliste

- 1: Vorrichtung
- 2: Behälter
- 2.1: Behälterwand
- 2.2: Elektroanschluss
- 2.3: Boden
- 2.4: Ablasshahn
- 3: Deckteil
- 3.1: Boden (von 3)
- 3.3: Kontaktteile
- 4: Trichteraufnahme
- 4.1: Rüttelantrieb
- 5: Trichter
- 5.1: Trichtermündung
- 6: Antriebswelle, Rührer
- 6.1: Lager
- 6.2: Antrieb
- 6.1, 6.3: Wellen
- 7: Rührbalken
- 7a: Rührbalken
- 7.1: Mittelteil
- 7.2: Außenteil
- 7.3: Lager
- 8: Flüssigkeit (Wasser)
- 9: Kühlung
- 9.1: Kühlgitter
- 9.2: Einlass
- 9.3: Auslass
- 10: Steuerung (Elektronik)
- 10.1: Bedienfeld
- 10.2: Lautsprecher
- 10.4: Temperatursensor
- 11: Heizung
- 12: Schaumzerstörer
- 12.1: Balken
- 12.2: Blatt
- 12.3: Kanten
- 13: Untersetzungsgetriebe
- 13.1: Zwischenwelle
- 13.2: Getriebegehäuse
- 13.3: Riemen
- 14: Elektroanschluss für externe Pumpe
- 21: Plattform
- 22: Schienenführung
- 23: Verbindungsteil
- 25: Siebeinheit
- 25, 1, 25.2: Siebe
- 25.3: Spannring
- 25.4: Siebhalterung
- 26: Schaumzerstörer
- 26.1: Scheibe
- 26.2: Durchbrechungen
- 26.3: Rotorblätter
- 26.4: innere Scheibe / Hauptkörper
- 26.5: äußerer Ring / Hauptkörper
- 26.6, 26.7: Stege
- 27: Montageplatte
- 27.1: Flansch
- 31: Dosiereinrichtung
- 31.1: Dosierscheibe
- 31.2, 31.3: Langlöcher / Dosierlöcher
- 31.4: Verbindungslöcher
- 32: Einlassscheibe
- 32.1: Einlassschlitze
- 33: Auslassscheibe
- 33.1: Auslassschlitze
- 34: Antriebsausgestaltung
- 34.1: Motorantrieb
- 34.2: Welle
- 34.3: Riemenscheibe
- 34.4: Zahnscheibe
- 34.5: Achse
- 34.6: Riemen- oder Zahnscheibe
- 34.7: Welle
- 34.8: Zahnscheibe
- 34.9: Zahnriemen
- 34.10: Zahnriemen

- A: Rotorachse

## Patentansprüche

1. Vorrichtung zum Rehydrieren von Mikroorganismen, mit einem Behälter (2) mit einer Temperaturregelung, mit einem eine in den Behälter (2) eingefüllte Flüssigkeit (8) rührenden Rührer (6) und mit einem Einfülltrichter (5) zum sukzessiven Einfüllen pulverförmiger Mikroorganismen in den Behälter (2).

2. Vorrichtung nach Anspruch 1, **gekennzeichnet durch** einen mit endlichem Abstand zur Flüssigkeitsoberfläche oberhalb derselben angeordneten drehbaren Schaumzerstörer (12), der vorzugsweise unmittelbar unterhalb eines Deckels (3) angeordnet ist.

3. Vorrichtung nach Anspruch 2, **dadurch gekennzeichnet, dass** der Schaumzerstörer (12) einzelne Rotorblätter (12a) aufweist, die vorzugsweise gegen die Ebene des Schaumzerstörers geneigt sind und damit unter einem endlichen Winkel ungleich 90° einer Drehachse des Schaumzerstörers ausgerichtet sind, wobei der Anstellwinkel zur Achse vorzugsweise zwischen 5° und 10° beträgt.

4. Vorrichtung nach einem der Ansprüche 1 bis 3, **gekennzeichnet durch** eine Dosiereinrichtung (31) zum Eindosieren der Mikroorganismen.

5. Vorrichtung nach Anspruch 4, **dadurch gekennzeichnet, dass** die Dosiereinrichtung (31) eine gelochte Dosierscheibe (31.1) und unmittelbar oberhalb sowie unterhalb derselben mit radial erstreckender Einlass- bzw. Auslassscheibe (32.1, 33.1) versehene Einlass- und Auslassscheiben (32, 33) aufweist.

6. Vorrichtung nach Anspruch 5, **dadurch gekennzeichnet, dass** die Schlitze (32.1, 33.1) der Einlass- und Auslassscheibe (32, 33) unter einem ersten Winkel α in Umfangsrichtung zueinander versetzt sind, die Schlitze (33.1) der Auslassscheibe (33) zu den Schlitzen (32.1) der Einlassscheibe (32) um einen zweiten Winkel β = α/2 in Umfangsrichtung zueinander versetzt sind und die Dosierscheibe (31.1) unter dem Winkel β zumindest in Umfangsrichtung zueinander versetzt Dosierlöcher (31.2, 31.3) aufweist.

7. Vorrichtung nach einem der Ansprüche 1 bis 6, **dadurch gekennzeichnet, dass** der Rührer (6) mindestens einen Rührbalken (7) aufweist, dass der oder die Rührbalken drehfest, aber einer der Rührbalken als Schwimmbalken axial verschieblich auf einer Antriebswelle gelagert ist bzw. sind.

8. Vorrichtung nach Anspruch 7, **dadurch gekennzeichnet, dass** zumindest ein Rührbalken (7) aus einem Material mit einer Dichte zwischen 0,9 und 0,95 g/cm³ besteht oder aus einem schwereren Rohr, gefüllt mit Styropor derart, dass die mittlere Dichte des Gesamtrohrs den vorgegebenen Wert hat.

9. Vorrichtung nach Anspruch 8, **dadurch gekennzeichnet, dass** der zumindest eine Rührbalken (7) aus Polypropylen oder Polyethylen besteht, dass vorzugsweise mindestens einer der Rührbalken beidseits eines Mittelteils (7.1) relativ zu diesem um ihre Achse drehbar gelagerte Drehabschnitte (7.2) aufweist und/oder dass vorzugsweise die Drehabschnitte (7.2) in ihrer Seitenwandung sich unter einem endlichen Winkel zur Drehachse erstreckende Nuten (der Mikroorganismen) aufweisen.

10. Vorrichtung nach Anspruch 9, **dadurch gekennzeichnet, dass** der Rührer zwei Rührbalken aufweist, von denen einer auf der Flüssigkeit schwimmt, ein weiterer axial festgehaltener in die Flüssigkeit eintaucht.

11. Vorrichtung nach Anspruch 9, **dadurch gekennzeichnet, dass** der Schaumzerstörer (12) vom gleichen Antrieb (6.2) wie der Rührer (7) angetrieben ist, und dass vorzugsweise Rührer (6) und/oder Schaumzerstörer (12) über ein Unter- oder Übersetzungsgetriebe (13) mit dem Antrieb (6.2) verbunden sind.

12. Vorrichtung nach einem der vorangehenden Ansprüche **dadurch gekennzeichnet, dass** ein Einfülltrichter (5) mit einem Rüttelantrieb (der Mikroorganismen) versehen ist, dessen Rüttelfrequenz vorzugweise einstellbar ist.

13. Verfahren zum Rehydrieren von Mikroorganismen, insbesondere unter Einsatz der erfindungsgemäßen Vorrichtung, wobei
- Flüssigkeit, entweder Wasser und/oder ein Most-Wasser-Gemisch im Behälter auf eine gewünschte Temperatur aufgeheizt wird,
- bei Erreichen der genannten Rehydrations-Temperatur ein akustisches Signal ausgegeben wird,
- anschließend bei sukzessivem rieselndem Einfüllen der Mikroorganismen, gegebenenfalls mit Mobilisator, in die im Behälter befindliche Flüssigkeit diese mittels eines langsam umlaufenden Rührers über eine vorgegebene Zeit, insbesondere die Einfüllzeit der Mikroorganismen umgerührt wird, um eine Klumpenbildung zu vermeiden,
- die Zieltemperatur über eine vorgegebene Zeit aufrecht erhalten wird, und
- anschließend unter Abschaltung der Heizeinrichtung wiederum ein akustisches Signal ausgegeben wird.

14. Verfahren nach Anspruch 13, **dadurch gekennzeichnet, dass** bei einem Most-Flüssigkeits-Gemisch, in welches die Trockenhefe eingegeben wird, entstehender Schaum durch einen schnell laufenden Schaumzerstörer, insbesondere in Form eines mit einer Kante versehenen Rotor zerteilt und damit zerstört wird.

15. Verfahren nach Anspruch 13 oder 14, **dadurch gekennzeichnet, dass** die erzeugte Mikroorganismen-Suspension nach Erreichen einer vorgegebenen Haltezeit auf ebenfalls vorgegebener erhöhter Temperatur mittels einer Kühleinrichtung auf eine zweite vorgegebene Temperatur abgekühlt wird und bei Erreichen der vorgewählten zweiten Temperatur ein akustisches Signal ausgegeben wird.

16. Verfahren nach einem der Ansprüche 13 bis 15, **dadurch gekennzeichnet, dass** das Suspensionsmaterial über zwei Siebe in den Behälter eingefüllt wird, dessen eines relativ zum anderen verschwenkt wird.

17. Verfahren nach einem der Ansprüche 13 bis 16, **dadurch gekennzeichnet, dass** eine erstellte Mikroorganismen-Suspension von einer Vorrichtung zur Erzeugung der Suspension mittels einer Pumpe in einen Most oder Maische enthaltenen Gärbehälter gepumpt wird.
